# FASCICULE DE BREVET EUROPEEN

(11) **EP 4 200 376 B1**
(45) Date de publication et mention de la délivrance du brevet: **01.07.2026**
(21) Numéro de dépôt: 21765644.6
(22) Date de dépôt: 19.08.2021
(51) Int. Cl.: C09J 133/14, A61L 24/04, C09J 143/02

(54) **COLLES CHIRURGICALES**
CHIRURGISCHER KLEBSTOFF
SURGICAL ADHESIVE

(30) Priorité: 20.08.2020 FR 2008594
(43) Date de publication de la demande: 28.06.2023
(73) Titulaire: Cohesives, 21000 Dijon (FR)
(72) Inventeur: PERRIN, Bertrand, 21000 DIJON (FR); GOUTAY, Natacha, 75018 PARIS (FR)
(74) Mandataire: Oudin, Stéphane
(86) Numéro de dépôt international: PCT/EP2021/073090
(87) Numéro de publication internationale: WO 2022/038247

(56) Documents cités:
- EP-A1- 3 514 213
- WO-A2-2008/082929
- US-A1- 2017 209 617

## Description

### Domaine technique de l'invention

La présente invention concerne notamment des compositions à utiliser comme adhésif chirurgical, produit d'étanchéification chirurgical, pansement hémostatique et/ou pansement cutané. Plus particulièrement, la présente invention concerne des compositions destinées à être utilisées dans un procédé:
- en tant qu'adhésif chirurgical pour l'adhésion d'un matériau à un tissu biologique,
- pour l'adhésion de tissus biologiques entre eux,
- pour l'adhésion d'une colle ou d'une substance à la surface d'un tissu biologique,
- en tant qu'étanchéificateur chirurgical,
- pour obturer ou colmater les orifices crées par une suture par fil ou par agrafe ou par une résection tissulaire (hémostase, aérostase, lymphostase par exemple),
- pour obturer un orifice, une incision ou une déchirure dans un tissu biologique,
- en tant qu'hémostatique pour arrêter un saignement, seul ou en complément techniques conventionnelles d'hémostase comme la suture, la compression ou l'électrocoagulation, ou
- en tant que pansement sur un tissu biologique pour recouvrir et protéger une plaie. Ces compositions peuvent également être utilisées
- pour renforcer un tissu biologique.
- pour fixer et stabiliser un tissu biologique.
- pour le traitement des lésions cutanées.

### Technique antérieure

Un certain nombre de techniques chirurgicales mettent en œuvre des colles chirurgicales. Ces dernières sont principalement utilisées pour aider à obtenir une hémostase chirurgicale. Toutefois, l'efficacité des colles chirurgicales dans cette indication est controversée et d'autres utilisations comme pour l'aérostase ne montrent pas de meilleurs résultats.

Par ailleurs, les colles chirurgicales ont de très faibles propriétés adhésives et ne peuvent donc pas être utilisées en tant qu'adhésif ni comme suture chirurgicale. L'application des colles chirurgicales se fait la plupart du temps directement sur le tissu, sans préparation de la surface de collage. La pénétration dans les tissus est faible ou inexistante ce qui entraine un collage de mauvaise qualité. Les déposants ont constaté que les colles actuelles ne collent pas et ne pénètrent pas dans les tissus.

Afin de répondre à ce problème, des colles chirurgicales de faibles viscosités ont été proposées. Ces colles chirurgicales pénètrent plus facilement dans les tissus ce qui permet d'obtenir un collage de meilleure qualité. Toutefois, ces colles chirurgicales nécessitent des concentrations importantes de monomères. Par ailleurs, leur nature chimique peut engendrer des brûlures sur le site d'apposition. Ces compositions sont également difficilement utilisables directement par le grand public.

En conséquence, la présente invention se propose de fournir un nouveau type de colles chirurgicales. Les compositions et le procédé selon l'invention permettent d'obtenir un niveau d'adhésion cutanée adapté à une application par le grand public. Les concentrations choisies permettant un amorçage rapide et une polymérisation en quelques dizaines de secondes, sans occasionner de brûlure des tissus.

US2017209617A1 divulgue une composition adhésive pour la liaison à des structures osseuses et similaires, comprenant du phosphate de bis [2(méthacryloyoxy) éthyl] (Bis-2), du phosphate de calcium modifié acrylique MCP, des monomères acryliques tels que HEMA, DMAEMA, UDMA, HMDMA et campherquinone comme photo amorceur pour l'adhésion sur des tissus minéralisés.

WO2008082929A2 divulgue une composition adhésive dentaire comprenant MDP (10 methacryloxydecyl phosphate), MHP (6 methacryloxyhexyl phosphate) ou MOP (8 methacryloxydecyl phosphate), des monomères acryliques et un oligomère d'acrylate de polyester durci avec des photoinitiateurs, par exemple la camphorquinone pour l'adhésion de tissus non minéralisés.

EP3514213A1 divulgue des compositions adhésives pour rubans médicaux d'esters phosphoriques à insaturation éthylénique, de monomères acryliques et d'initiateur peroxy pivalate.

Par ailleurs, les résines photopolymérisables choisies, combinées aux monomères polymérisables, apportent au collage obtenu des propriétés de flexibilité, une bonne stabilité dans le temps sous l'action des liquides physiologiques (sang exsudats, transpiration), une limitation de l'accumulation de transpiration ou d'exsudats à l'interface tissus/collage et une bonne résistance à l'eau.

### Résumé de l'invention

La présente invention concerne notamment des compositions pour utilisation en tant qu'adhésif chirurgical pour l'adhésion d'un matériau à un tissu biologique non minéralisé, pour l'adhésion de tissus biologiques non minéralisés entre eux, pour l'adhésion d'une colle ou d'une substance à la surface d'un tissu biologique non minéralisé, en tant qu'étanchéificateur chirurgical, pour obturer ou colmater les orifices crées par une suture par fil ou par agrafe ou par une résection tissulaire, pour obturer un orifice, une incision ou une déchirure dans un tissu biologique non minéralisé, en tant qu'hémostatique pour arrêter un saignement, en tant que pansement sur un tissu biologique non minéralisé pour recouvrir et protéger une plaie, pour renforcer un tissu biologique non minéralisé, pour fixer et stabiliser un tissu biologique non minéralisécomprenant :
- un monomère polymérisable comprenant une fonction phosphate ou phosphonate d'une part et une fonction méthacrylate, acrylate, acrylamide ou methacrylamide d'autre part à une concentration comprise entre 5 et 40% en masse par rapport à la masse totale de la composition,
- un amorceur de polymérisation à une concentration comprise entre 0,5 et 2% en masse, et,
- une résine photopolymérisable.

Dans le cadre de la présente invention, le monomère polymérisable comprenant une fonction phosphate ou phosphonate d'une part et une fonction méthacrylate, acrylate, acrylamide ou methacrylamide d'autre part pourra être désigné « monomère polymérisable ».

Les déposants ont pu mettre en évidence que les compositions selon présentaient des capacités adhésives et une innocuité supérieure aux compositions de l'art antérieur. Ces compositions sont donc particulièrement adaptées pour une utilisation cutanée comme pansement pour les blessures et/ou pour le traitement de toutes lésions cutanées.

Préférentiellement, ledit monomère polymérisable est choisi dans le groupe consistant en les molécules ayant le numéro CAS [14206-25-8], [14235-57-5], [86242-61-7], [932019-41-6], [1980781-17-6], [60161-88-8], [87243-97-8], [1980048-95-0], [918802-80-9], [63411-25-6], [1980064-07-0], [22432-83-3], [1980781-08-5], [252210-28-9], [1114567-37-1], [80730-17-2], [518991-74-7], [87243-96-7], [1980062-84-7], [518991-75-8], [252210-30-3], [22432-84-4], [727415-30-7], [727415-31-8], [784139-89-5] ou [1194231-98-5] et leurs mélanges.

Préférentiellement, le monomère polymérisable est de formule I 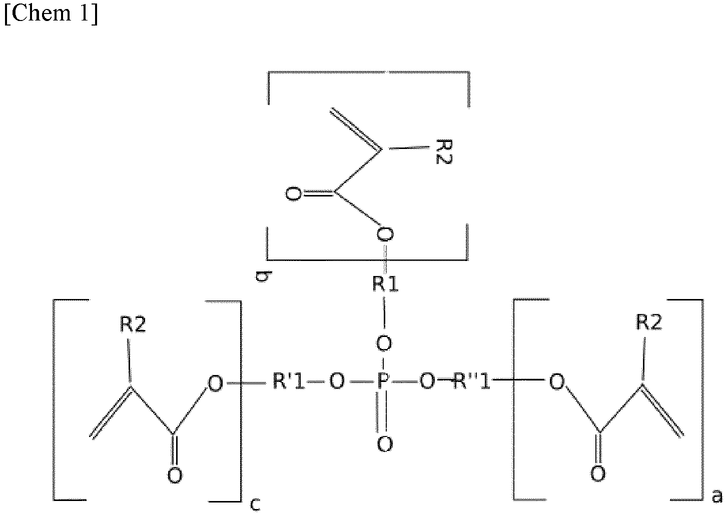 pour laquelle
R2 est H ou CH3;
R1, R1', R1'' sont indépendamment les uns des autres un radical polyether linéaire, un radical aliphatique linéaire ou ramifié en C1-C50, un radical aromatique en C6-C18 , pour lesquels la chaîne carbonée desdits radicaux peut être interrompue par O, S, OCONH et/ou peut comprendre une ou plusieurs fonctions alcool;
R1' est H si c=0;
R1" est H si a=0;
b est 1, ;
a ou c est 1 ou 0.

Le terme « la chaîne carbonée desdites fonctions peut être interrompue » entend signifier que lesdites fonctions sont insérées dans la chaîne carbonée, i.e. sont liés à des atomes de carbone des deux côtés.

Préférentiellement, a=0, R2=H ou CH3 et R'1 et R1 est une chaîne aliphatique linéaire en C1-C12.

Encore plus préférentiellement, a=0, c=0, R2=CH3 et R1 est une chaîne aliphatique linéaire en C1-C12.

Préférentiellement, a=0, c=0, R"1=H, R'1=H, R1=chaîne aliphatique linéaire en C1-C12, b = 1, R2 = CH3.

Préférentiellement, a=1, c=1, b=0, R1=H, R'1=R''1=chaîne aliphatique linéaire en C1-C12, R2=CH3.

Préférentiellement, le monomère polymérisable de formule I est le 10-MDP (C14H27O6P, numéro CAS [85590-00-7]) ou le MEP(C12H19O8P, numéro CAS [32435-46-4]).

Selon un autre mode de réalisation préféré, le monomère polymérisable de formule I est choisi parmi le glycérol diméthacrylate phosphate, l'éthylène glycol méthacrylate phosphate, le polyéthylène glycol méthacrylate phosphate, le méthacryloyloxy décyl hydrogène phosphate, le méthacryloyloxy l'éthyloxy hydrogène phosphate, le glycérol monométhacrylate phosphate, le triéthylene glycol monomethacrylate phosphate, methacryloyloxy propyl phosphate, methacryloyloxy hexyl phosphate, methacrylated aminoethyl phosphonic acid, bis(glyceryl dimethacrylate) phosphate et leurs mélanges.

Dans le cadre de la présente invention le terme « monomère polymérisable » entend désigner un monomère dont la polymérisation peut être initiée par un amorceur physique ou chimique.

Selon un mode de réalisation préféré, la polymérisation est initiée sous l'effet d'un rayonnement. De manière préférée, ledit rayonnement a une longueur d'onde comprise entre de 350 nm à 520 nm.

Le polymère obtenu après polymérisation du monomère est préférentiellement un polymère biocompatible.

Selon un mode de réalisation préféré, ladite viscosité est inférieure à 120 Pa.s à 20°C.

Selon un mode de réalisation encore plus préféré, ladite viscosité est inférieure à 107 Pa.s à 20°C.

Selon un mode de réalisation tout à fait préféré, ladite viscosité est inférieure à 50 Pa.s à 20°C.

Selon un mode de réalisation tout à fait préféré, ladite viscosité est supérieure à 20 Pa.s à 20°C.

La viscosité de la composition peut notamment être mesurée par un viscosimètre à chute de bille selon la norme DIN53015.

Selon un mode de réalisation préféré, la composition selon l'invention n'est pas un hydrogel.

Selon un mode de réalisation préféré, ledit monomère a une masse molaire comprise entre 250 et 500g.mol-1.

Selon un mode de réalisation encore plus préféré, ledit monomère a une concentration comprise entre 20 et 40% en masse par rapport à la masse totale de la composition.

Selon un mode de réalisation encore plus préféré, ledit monomère a une concentration comprise entre 20 et 40% en masse par rapport à la masse totale de la composition.

Selon un mode de réalisation tout à fait préféré, ledit monomère a une concentration comprise entre 25 et 35% en masse par rapport à la masse totale de la composition.

Selon un mode de réalisation préféré de l'invention, ladite résine photopolymérisable est choisie dans le groupe consistant en les résines uréthanes acrylates aliphatiques, les résines uréthanes acrylates hydrophobes, les résines uréthanes acrylates aromatiques, les résines polyéthers uréthanes acrylates, et leurs mélanges.

Selon un mode de réalisation préféré de l'invention, ladite résine uréthane acrylate aliphatique est choisie dans le groupe consistant en Allnex Ebecryl 230^{®}, Allnex IRR 907^{®} Allnex Ebecryl 4491^{®}, Allnex Ebecryl 1271^{®} et leurs mélanges.

Selon un mode de réalisation préféré de l'invention, ladite résine uréthane acrylate aromatique est le Allnex Ebecryl 210^{®}.

Selon un mode de réalisation préféré de l'invention, ladite résine uréthane acrylate hydrophobe est le Dymax Bomar BRC-843 S^{®}.

Selon un mode de réalisation préféré de l'invention, ladite résine polyéther uréthane acrylate est le Dymax Bomar BR-3641AJ^{®}.

Les différentes résines identifiées ci-dessus donne au collage obtenu avec la composition selon l'invention des propriétés de flexibilité optimales tout en limitant l'exothermie de la réaction de polymérisation à un niveau acceptable.

Selon un autre mode de réalisation préféré, ladite composition comprend entre 50% et 90% encore plus préférentiellement entre 60 et 80%, en masse par rapport à la masse totale de la composition, de ladite résine photopolymérisable.

Selon un mode de réalisation préféré la composition selon l'invention comprend un amorceur de polymérisation et encore plus préférentiellement un photoamorceur. L'utilisation d'un amorceur de polymérisation thermique ou redox n'est pas exclue du cadre de la présente invention. Parmi les amorceurs redox utilisables, on peut notamment citer le couple dibenzoyle peroxide/amine (trimethylaniline).

Selon un mode de réalisation encore plus préféré ledit photoamorceur est capable d'induire une polymérisation sous l'effet d'une rayonnement compris entre 350 et 520nm.

Ledit photoamorceur est préférentiellement choisie parmi : l'oxyde de 2,4,6-triméthylbenzoyl-phénylphosphinate (TPO-L), la camphorquinone ou 4,4'-bis(diethylamino)benzophenone cette dernière associée à l'Ethyl-4-(dimethylamino)benzoate (EDB), et leurs mélanges.

Selon un autre mode de réalisation préféré, ladite composition est dépourvue de solvant.

Selon un mode réalisation préféré de l'invention, ladite composition comprend entre 0.5 et 2% en masse dudit photoamorceur, entre 50 et 90% en masse de ladite résine photopolymérisable, entre 20 et 40% en masse de monomère polymérisable.

Selon un mode réalisation tout à fait préféré de l'invention, ladite composition comprend entre 0.5 et 2% en masse de TPO-L ou de camphorquinone associée à l'EDB, entre 50 et 90% en masse de ladite résine uréthane acrylate aliphatique, entre 20 et 40% en masse de MDP ou de MEP.

Dans le cadre de la présente invention, lorsque les concentrations des différents composants de la composition selon l'invention sont indiquées en pourcentage il s'agit du pourcentage en masse dudit composant par rapport à la masse totale de ladite composition.

Dans le cadre de la présente invention, le terme « comprend » entend signifier que la composition selon l'invention inclut les éléments cités. De manière préférée, la présente invention concerne des compositions comprenant uniquement les éléments cités à l'exclusion de tout autre.

La composition selon l'invention peut être utilisée dans un procédé remarquable en ce qu'il comprend les étapes :
- (i) enduire le tissu à traiter d'une composition selon invention,
- (ii) laisser pénétrer la composition dans ledit tissu,
- (iii) induire la polymérisation de ladite composition.

Selon un mode de réalisation de l'invention, l'étape (ii) est optionelle.

Dans le cadre de la présente invention, le terme « tissu biologique » entend désigner préférentiellement les tissus biologiques non minéralisés.

Par souci de clarté, il est précisé que, dans le cadre de la présente invention, le terme « tissus biologique » n'entend pas désigner les os et les dents.

Le procédé est préférentiellement non-invasif. Le terme « non-invasif » entend signifier que le procédé selon l'invention ne comprend aucune étape chirurgicale consistant à accéder au tissu à traiter. Ainsi, le procédé selon l'invention est mis en œuvre sur un tissu biologique directement accessible (e.g. la peau) ou préalablement rendu accessible par d'autres méthodes.

Le procédé est préférentiellement un procédé non-invasif pour recouvrir et protéger une lésion cutanée.

Le procédé est préférentiellement un procédé non-invasif pour rapprocher les lèvres d'une plaie cutanée.

Alternativement, le procédé est un procédé pour l'adhésion d'un matériau à un tissu biologique, pour l'adhésion de tissus biologiques entre eux, pour l'adhésion d'une colle ou d'une substance à la surface d'un tissu biologique, l'étanchéification chirurgical, pour obturer ou colmater les orifices crées par une suture par fil ou par agrafe ou par une résection tissulaire (hémostase, aérostase, lymphostase par exemple), pour obturer un orifice une incision ou une déchirure dans un tissu biologique, pour arrêter un saignement, pour recouvrir et protéger une plaie, pour renforcer un tissu biologique ou pour fixer et stabiliser un tissu biologique.

Par « cutanée », on entend désigner une localisation située sur la peau, les lèvres ou la muqueuse buccale.

Selon un mode de réalisation préféré, ladite étape (iii) est réalisée à l'aide d'un rayonnement UV ou de lumière visible. Les caractéristiques du rayonnement mis en œuvre, notamment sa puissance et sa longueur d'onde, sont adaptées aux constituants de la composition, notamment à la nature du monomère polymérisable et à la nature de l'amorceur de polymérisation.

La composition selon l'invention peut également être comprise dans un ensemble de parties comprenant une composition selon l'invention et une source de rayonnement

De préférence la source de rayonnement de l'ensemble de parties peut émettre un rayonnement adapté pour polymériser et/ou aider la polymérisation et/ou accélérer la polymérisation du monomère polymérisable de la composition.

Dans le cadre de la présente invention, le terme « source de rayonnement » fait référence à tout moyen artificiel apte à produire un rayonnement de longueur d'onde comprise entre 350 et 520. De manière préférée ledit rayonnement UV est une puissance d'irradiance comprise entre 10mW/cm2 et 100 mW/cm2 et encore plus préférentiellement entre 17mW/cm2 et 92 mW/cm2.

### Description des modes de réalisation

### Test de réaction cutanée chez l'homme

Des compositions selon l'invention ont été déposées sur la paume. La composition est déposée à la surface de la peau puis polymérisée par un rayonnement UV.

La présence ou l'absence de réactions cutanées sont observées lors de la mise en place puis dans les jours qui suivent.

### Optimisation de la concentration en photoamorceur

Des tests ont été effectués avec des compositions selon l'invention comprenant 30% de MEP, 69.5% d'Ebecryl 230 et des concentrations variables de TPO-L.

On observe, pour des concentrations en photoamorceur inférieures à 0.5%, une faible tenue dans le temps des collages et un allongement du temps de photopolymérisation (>20s) incompatible avec une utilisation de la composition.

On observe également, pour des concentrations supérieures à 2%, une exothermie de polymérisation incompatible avec une utilisation in vivo des compositions.

### Optimisation de l'irradiance de photopolymérisation I0

Des tests ont été effectués avec des compositions selon l'invention comprenant 30% de MEP, 69.5% d'Ebecryl 230 et 1% TPOL.

Ces compositions ont été utilisés suivant le protocole précédemment décrit et soumises à un rayonnement avec une source UV à 395nm avec des irradiances variables

La borne inférieure de l'irradiance (17mW/cm2) est limitée par la cinétique de polymérisation et l'inhibition par l'oxygène de la réaction. La borne supérieure d'irradiance (92mW/cm2) est limitée par l'exothermie de réaction. Toutefois des valeurs d'irradiance entre 10 et 100 mW/cm2 offrent des résultats acceptables.

### Efficacité des compositions selon l'invention

L'évaluation qualitative de l'adhésion In Vivo et de la qualité du collage a été évalué sur de la peau humaine (N = 1 à 3 tests). Une échelle d'adhésion qualitative a été mise au point :
0 : pas d'adhésion à t = 0, aucun ancrage
1 : adhésion à t = 0, très faible ancrage, décollement en quelques minutes, aucune résistance à la sollicitation
2 : adhésion à t = 0, faible ancrage, tenue < 1h, pas de résistance à la sollicitation, décollement rapide sur les bords
3 : adhésion à t = 0, faible ancrage, tenue 2h à 4h, faible résistance à la sollicitation, décollement rapide sur les bords
4 : adhésion à t = 0, faible ancrage, tenue 4h à 8h, faible résistance à la sollicitation, décollement rapide sur les bords
5 : adhésion à t = 0, ancrage moyen, tenue 4h à 8h, résistance à la sollicitation moyenne, résistance au décollement sur les bords
6 : adhésion à t = 0, ancrage moyen, tenue 8h à 12h, résistance à la sollicitation moyenne, résistance au décollement sur les bords
7 : adhésion à t = 0, ancrage moyen, tenue 12h à 24h, résistance à la sollicitation bonne à moyenne, bonne résistance au décollement sur les bords
8 : adhésion à t = 0, bon ancrage, tenue 24h à 36h, bonne résistance à la sollicitation, bonne résistance au décollement sur les bords
9 : adhésion à t = 0, bon ancrage, tenue 36h à 48h, très bonne résistance à la sollicitation, bonne résistance au décollement sur les bords
10 : adhésion à t = 0, excellent ancrage, tenue > 48h, excellente résistance à la sollicitation, pas de décollement aux bords.

L'ensemble des résultats obtenus avec la combinaison TPO-L + MEP + Ebecryl 230 est présenté dans le Tableau 1.

Un traitement statistique de ces données a mis en évidence une influence majeure de la concentration en photoamorceur et en monomère adhésif sur les propriétés d'adhésion et de tenue. Plus ces concentrations sont élevées, plus les propriétés adhésives augmentent. Au contraire, de faibles concentrations conduisent à des niveaux d'adhésion et de tenue plus faibles. L'irradiance de la source UV LED (à 399nm) influence également les propriétés adhésives des collages. Par ailleurs, la sensibilité à l'eau de chacune des couches d'adhésion obtenue a été compatible avec l'utilisation des compositions dans le procédé selon l'invention.

**[Tableau 1]**

| [TPOL] %wt | [MEP] %wt | [Ebecryl 230] %wt | I0 (mW/cm²) | Tenue In Vivo | Pelage 90° | Temps de @ | Exothermie | Flexibilité |
|---|---|---|---|---|---|---|---|---|
| 1 | 30 | 69 | 17 à 20 | 8 | 6,2 | < 20s | +++ | ++ |
| 0,5 | 20 | 79 | 17 à 20 | 3 | N/D | < 20s | 0 | +++ |
| 0,5 | 20 | 79 | 92 | 4 | 1,1 | < 20s | 0 | ++ |
| 0,5 | 40 | 59 | 17 à 20 | 7 | N/D | < 20s | 0 | ++ |
| 0,5 | 40 | 59 | 92 | 6 | 2,3 | < 20s | 0 | + |
| 2 | 20 | 79 | 17 à 20 | 6 | N/D | < 20s | + | +++ |
| 2 | 20 | 79 | 92 | 7 | 0,8 | < 20s | + | ++ |
| 2 | 40 | 59 | 17 à 20 | 8 | N/D | < 20s | +++ | + |

| | | | | | | | | |
|---|---|---|---|---|---|---|---|---|
| [Tableau 1] représente les résultats expérimentaux sur la formulation TPOL + MEP + Ebecryl 230. | | | | | | | | |

Les résultats obtenus dans le tableau 2 montrent que La concentration en monomère adhésif peut également être abaissée à 5%.

**[Tableau 2]**

| [TPOL] %wt | [MEP] %wt | [Ebecryl 230] %wt | I0 (mW/cm²) | Tenue In Vivo | Temps de @ | Exothermie | Flexibilité |
|---|---|---|---|---|---|---|---|
| 1 | 30 | 69 | 17 à 20 | 8 | < 20s | +++ | ++ |
| 0,25 | 40 | 59,75 | 20 | 2 | 20s < t_{@} < 40s | + | + |
| 0,1 | 40 | 59,9 | 20 | 2 | 40s < t_{@} < 60s | + | ++ |
| 0,5 | 30 | 69,5 | 20 | 5 | < 20s | 0 | ++ |
| 1 | 25 | 74 | 20 | 8 | < 20s | +++ | ++ |
| 1 | 5 | 94 | 20 | 1 | < 20s | 0 | +++ |
| 1 | 10 | 89 | 20 | 2 | < 20s | 0 | +++ |
| 1 | 20 | 79 | 20 | 2 | < 20s | + | ++ |

| | | | | | | | |
|---|---|---|---|---|---|---|---|
| [Tableau 2] représente les résultats expérimentaux complémentaires sur la formulation TPOL + MEP + Ebecryl 230. | | | | | | | |

## Revendications

1. Composition pour utilisation en tant qu'adhésif chirurgical pour l'adhésion d'un matériau à un tissu biologique non minéralisé, pour l'adhésion de tissus biologiques non minéralisés entre eux, pour l'adhésion d'une colle ou d'une substance à la surface d'un tissu biologique non minéralisé, en tant qu'étanchéificateur chirurgical, pour obturer ou colmater les orifices crées par une suture par fil ou par agrafe ou par une résection tissulaire, pour obturer un orifice, une incision ou une déchirure dans un tissu biologique non minéralisé, en tant qu'hémostatique pour arrêter un saignement, en tant que pansement sur un tissu biologique non minéralisé pour recouvrir et protéger une plaie, pour renforcer un tissu biologique non minéralisé, pour fixer et stabiliser un tissu biologique non minéralisé comprenant :
- un monomère polymérisable comprenant une fonction phosphate ou phosphonate d'une part et une fonction méthacrylate, acrylate, acrylamide ou methacrylamide d'autre part à une concentration comprise entre 5 et 40% en masse par rapport à la masse totale de la composition,
- un photoamorceur à une concentration comprise entre 0,5 et 2% en masse, et
- une résine photopolymérisable.

2. Composition selon la revendication précédente **caractérisée en ce que** ledit monomère polymérisable est choisi dans le groupe consistant en les molécules ayant le numéro CAS [14206-25-8], [14235-57-5], [86242-61-7], [932019-41-6], [1980781-17-6], [60161-88-8], [87243-97-8], [1980048-95-0], [918802-80-9], [63411-25-6], [1980064-07-0], [22432-83-3], [1980781-08-5], [252210-28-9], [1114567-37-1], [80730-17-2], [518991-74-7], [87243-96-7], [1980062-84-7], [518991-75-8], [252210-30-3], [22432-84-4], [727415-30-7], [727415-31-8], [784139-89-5] ou [1194231-98-5] et leurs mélanges.

3. Composition selon la revendication 1 **caractérisée en ce que** ledit monomère polymérisable est de formule I 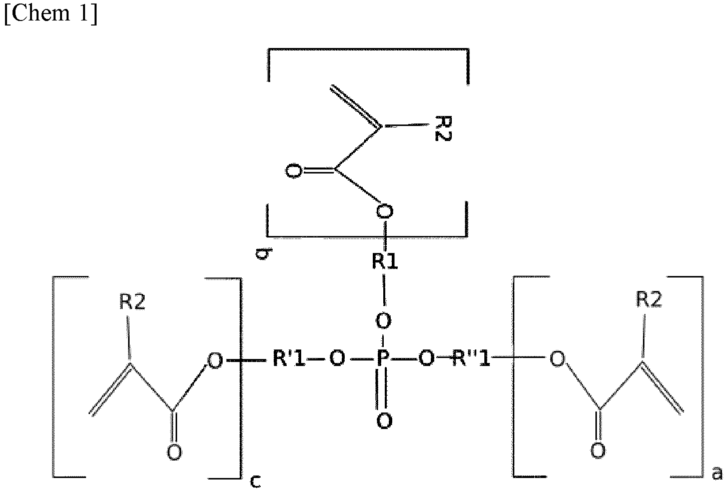 pour laquelle
R2 est H ou CH3;
R1, R1', R1'' sont indépendamment les uns des autres un radical polyether linéaire, un radical aliphatique linéaire ou ramifié en C1-C50, un radical aromatique en C6-C18 , pour lesquels la chaîne carbonée desdits radicaux peut être interrompue par O, S, OCONH et/ou peut comprendre une ou plusieurs fonctions alcool;
R1' est H si c=0;
R1" est H si a=0;
b est 1, ;
a ou c est 1 ou 0.

4. Composition selon la revendication précédente **caractérisée en ce que** a=0, R2=H ou CH3 et R'1 et R1 est un chaîne aliphatique linéaire en C1-C12.

5. Composition selon la revendication précédente **caractérisée en ce que** a=0, c=0, R2=CH3 et R1 est un chaîne aliphatique linéaire en C1-C12.

6. Composition selon la revendication 1 **caractérisée en ce que** le monomère polymérisable de formule I est le 10-MDP (C14H27O6P) de numéro CAS [85590-00-7] ou le MEP(C12H1 9O8P) de numéro CAS [32435-46-4].

7. Composition selon l'une des revendications précédentes, **caractérisée en ce que** ledit monomère polymérisable a une concentration comprise entre 25 et 35% en masse par rapport à la masse totale de la composition.

8. Composition selon l'une des revendications précédentes, **caractérisée en ce que** ledit photoamorceur est capable d'induire une polymérisation sous l'effet d'une rayonnement compris entre 350 et 520nm.

9. Composition selon l'une des revendications précédentes, **caractérisée en ce que** ledit photo-amorceur est choisi dans le groupe constitué de l'oxyde de 2,4,6-triméthylbenzoyl-phénylphosphinate (TPO-L), la camphorquinone ou 4,4'-bis(diethylamino)benzophenone cette dernière associée l'Ethyl-4-(dimethylamino)benzoate (EDB), et leurs mélanges.

10. Composition selon l'une des revendications précédentes **caractérisée en ce qu'**elle comprend entre 0.25 et 2% en masse dudit photoamorceur, entre 50 et 90% en masse de ladite résine photopolymérisable et entre 20 et 40% en masse dudit monomère polymérisable.

## Patentansprüche

1. Zusammensetzung zur Verwendung als chirurgischer Klebstoff zur Haftung von Material an nicht mineralisiertem biologischem Gewebe, zur Haftung von nicht mineralisiertem biologischem Gewebe untereinander, zur Haftung eines Klebstoffs oder einer Substanz an der Oberfläche von nicht mineralisiertem biologischem Gewebe, als chirurgisches Dichtungsmittel, zum Verschließen oder Abdichten von Öffnungen, die durch Faden- oder Klammernaht oder durch Geweberesektion entstanden sind, zum Verschließen einer Öffnung, einer Inzision oder eines Risses in nicht mineralisiertem biologischem Gewebe, als Hämostasemittel zum Stoppen von Blutungen, als Verband auf nicht mineralisiertem biologischem Gewebe, um eine Wunde abzudecken und zu schützen, zur Verstärkung von nicht mineralisiertem biologischem Gewebe, zur Fixierung und Stabilisierung von nicht mineralisiertem biologischem Gewebe, einschließlich:
- ein polymerisierbares Monomer, das einerseits eine Phosphat- oder Phosphonatfunktion und andererseits eine Methacrylat-, Acrylat-, Acrylamid- oder Methacrylamidfunktion in einer Konzentration zwischen 5 und 40 Massenprozent, bezogen auf die Gesamtmasse der Zusammensetzung, umfasst,
- einen Photoinitiator mit einer Konzentration zwischen 0,5 und 2 Massenprozent, und
- ein lichthärtendes Harz.

2. Zusammensetzung nach dem vorhergehenden Anspruch, **dadurch gekennzeichnet, dass** das polymerisierbare Monomer aus der Gruppe ausgewählt ist, bestehend aus den Molekülen mit der CAS-Nummer [14206-25-8], [14235-57-5], [86242-61-7], [932019-41-6], [1980781-17-6], [60161-88-8], [87243-97-8], [1980048-95-0], [918802-80-9], [63411-25-6], [1980064-07-0], [22432-83-3], [1980781-08-5], [252210-28-9], [1114567-37-1], [80730-17-2], [518991-74-7], [87243-96-7], [1980062-84-7], [518991-75-8], [252210-30-3], [22432-84-4], [727415-30-7], [727415-31-8], [784139-89-5] oder [1194231-98-5] und deren Mischungen.

3. Zusammensetzung nach Anspruch 1, **dadurch gekennzeichnet, dass** das polymerisierbare Monomer die Formel I aufweist 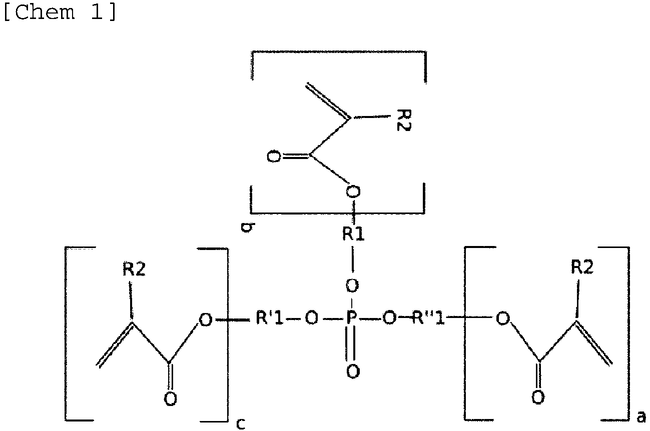 wobei
R2 H oder CH3 ist;
R1, R1', R1" unabhängig voneinander ein lineares Polyetherradikal, ein lineares oder verzweigtes aliphatisches C1-C50-Radikal, ein aromatisches C6-C18-Radikal sind, wobei die Kohlenstoffkette der Radikale durch O, S, OCONH unterbrochen sein kann und/oder eine oder mehrere Alkoholfunktionen umfassen kann;
R1' H ist, wenn c=0;
R1" H ist, wenn a=0;
b 1 ist;
a oder c 1 oder 0 ist.

4. Zusammensetzung nach dem vorhergehenden Anspruch, **dadurch gekennzeichnet, dass** a=0, R2=H oder CH3 und R'1 und R1 eine lineare aliphatische C1-C12-Kette ist.

5. Zusammensetzung nach dem vorhergehenden Anspruch, **dadurch gekennzeichnet, dass** a=0, c=0, R2=CH3 und R1 eine lineare aliphatische C1-C12-Kette ist.

6. Zusammensetzung nach Anspruch 1, **dadurch gekennzeichnet, dass** das polymerisierbare Monomer der Formel I 10-MDP (C14H27O6P) mit der CAS-Nummer [85590-00-7] oder MEP(C12H1 9O8P) mit der CAS-Nummer [32435-46-4] ist.

7. Zusammensetzung nach einem der vorhergehenden Ansprüche, **dadurch gekennzeichnet, dass** das polymerisierbare Monomer eine Konzentration zwischen 25 und 35 Massenprozent, bezogen auf die Gesamtmasse der Zusammensetzung, aufweist.

8. Zusammensetzung nach einem der vorhergehenden Ansprüche, **dadurch gekennzeichnet, dass** der Photoinitiator unter der Wirkung einer Strahlung zwischen 350 und 520 nm eine Polymerisation induzieren kann.

9. Zusammensetzung nach einem der vorhergehenden Ansprüche, **dadurch gekennzeichnet, dass** der Photoinitiator aus der Gruppe ausgewählt ist, die aus 2,4,6-Trimethylbenzoyl-phenylphosphinatoxid (TPO-L), Campherchinon oder 4,4'-Bis(diethylamino)benzophenon, wobei letzteres mit Ethyl-4-(dimethylamino)benzoat (EDB) verbunden ist, und deren Mischungen besteht.

10. Zusammensetzung nach einem der vorhergehenden Ansprüche, **dadurch gekennzeichnet, dass** sie zwischen 0,25 und 2 Massenprozent des Photoinitiators, zwischen 50 und 90 Massenprozent des photopolymerisierbaren Harzes und zwischen 20 und 40 Massenprozent des polymerisierbaren Monomers umfasst.

## Claims

1. Composition for use as a surgical adhesive for the adhesion of a material to a non-mineralised biological tissue, for the adhesion of non-mineralised biological tissues to one another, for the adhesion of a glue or a substance at the surface of a non-mineralised biological tissue, as a surgical sealant, to seal or plug orifices created by wire or staple suture or by tissue resection, to seal an orifice, an incision or a tear in a non-mineralised biological tissue, as a haemostatic to stop bleeding, as a dressing on a non-mineralised biological tissue to cover and protect a wound, to reinforce a non-mineralised biological tissue, to fix and stabilise a non-mineralised biological tissue comprising:
- a polymerisable monomer comprising a phosphate or phosphonate function on the one hand and a methacrylate, acrylate, acrylamide or methacrylamide function on the other hand at a concentration comprised between 5 and 40% by weight relative to the total weight of the composition,
- a photoinitiator at a concentration comprised between 0.5 and 2% by weight, and
- a photopolymerisable resin.

2. Composition according to the preceding claim, **characterised in that** said polymerisable monomer is selected from the group consisting of molecules having the CAS number [14206-25-8], [14235-57-5], [86242-61-7], [932019-41-6], [1980781-17-6], [60161-88-8], [87243-97-8], [1980048-95-0], [918802-80-9], [63411-25-6], [1980064-07-0], [22432-83-3], [1980781-08-5], [252210-28-9], [1114567-37-1], [80730-17-2], [518991-74-7], [87243-96-7], [1980062-84-7], [518991-75-8], [252210-30-3], [22432-84-4], [727415-30-7], [727415-31-8], [784139-89-5] or [1194231-98-5] and mixtures thereof.

3. Composition according to claim 1, **characterised in that** said polymerisable monomer is of formula I 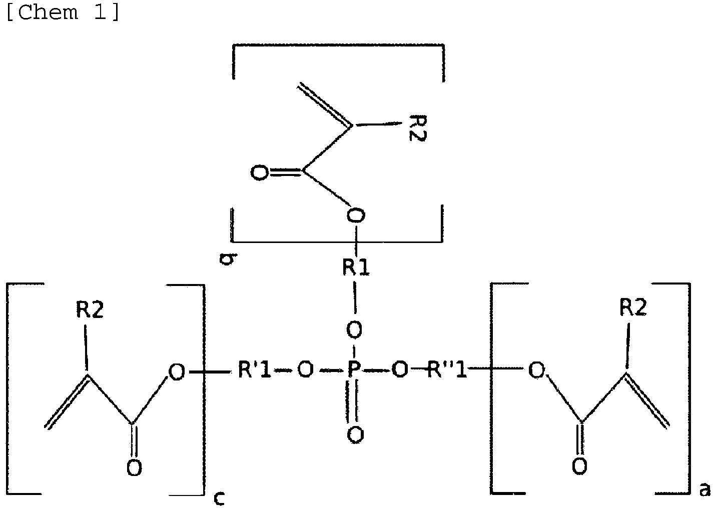 wherein
R2 is H or CH3;
R1, R1', R1'' are independently of each other a linear polyether radical, a linear or branched C1-C50 aliphatic radical, a C6-C18 aromatic radical, wherein the carbon chain of said radicals may be interrupted by O, S, OCONH and/or may comprise one or more alcohol function(s);
R1' is H if c=0;
R1" is H if a=0;
b is 1;
a or c is 1 or 0.

4. Composition according to the preceding claim, **characterised in that** a=0, R2=H or CH3 and R'1 and R1 is a linear C1-C12 aliphatic chain.

5. Composition according to the preceding claim, **characterised in that** a=0, c=0, R2=CH3 and R1 is a linear C1-C12 aliphatic chain.

6. Composition according to claim 1, **characterised in that** the polymerisable monomer of formula I is 10-MDP (C14H27O6P) of CAS number [85590-00-7] or MEP (C12H19O8P) of CAS number [32435-46-4].

7. Composition according to one of the preceding claims, **characterised in that** said polymerisable monomer has a concentration comprised between 25 and 35% by weight relative to the total weight of the composition.

8. Composition according to one of the preceding claims, **characterised in that** said photoinitiator is capable of inducing a polymerisation under the effect of radiation comprised between 350 and 520 nm.

9. Composition according to one of the preceding claims, **characterised in that** said photoinitiator is selected from the group consisting of 2,4,6-trimethylbenzoylphenylphosphinate oxide (TPO-L), camphorquinone or 4,4'-bis(diethylamino)benzophenone, the latter associated with Ethyl-4-(dimethylamino)benzoate (EDB), and mixtures thereof.

10. Composition according to one of the preceding claims, **characterised in that** it comprises between 0.25 and 2% by weight of said photoinitiator, between 50 and 90% by weight of said photopolymerisable resin and between 20 and 40% by weight of said polymerisable monomer.
